# EUROPEAN PATENT SPECIFICATION

(11) **EP 3 570 920 B1**
(45) Date of publication and mention of the grant of the patent: **23.03.2022**
(21) Application number: 18702152.2
(22) Date of filing: 18.01.2018
(51) Int. Cl.: A61M 16/08, A61M 39/10, A61M 39/12, F16L 33/00, A61M 16/06

(54) **PATIENT INTERFACE CONNECTOR**
PATIENTENSCHNITTSTELLENVERBINDER
CONNECTEUR D'INTERFACE PATIENT

(30) Priority: 20.01.2017 GB 201701042
(43) Date of publication of application: 27.11.2019
(60) Divisional application: 21199981.8
(73) Proprietor: Intersurgical AG, 9490 Vaduz (LI)
(72) Inventor: BOWSHER, Richard Francis, Wokingham Berkshire RG41 2RZ (GB)
(74) Representative: Adamson Jones
(86) International application number: PCT/EP2018/051241
(87) International publication number: WO 2018/134321

(56) References cited:
- WO-A1-2014/124323
- WO-A1-2015/033263
- US-A1- 2014 261 433
- US-B2- 7 174 893

## Description

The present invention relates to a patient interface connector for connecting a patient interface to a conduit.

Oxygen delivery masks are used to supply a patient with oxygen, to aid their normal respiratory function. In general, oxygen is supplied to a respiratory enclosure defined by the oxygen delivery mask and the face of the patient, and the patient inhales the oxygen from this respiratory enclosure, sometimes along with ambient gases from outside the respiratory enclosure. Conventional oxygen delivery masks typically have an inlet for the oxygen, as well as inhalation and exhalation outlets through which ambient gases and exhaled gases can pass.

To connect a supply of oxygen to the inlet of the mask, it is typical to utilise a connector which is connected to the inlet of the oxygen delivery mask via a friction fit connection. Whilst this can provide an adequate connection between the oxygen delivery mask and the supply of oxygen, such connections can lead to misconnection of other forms of medical tubing, for example feeding tubing, to the oxygen delivery mask, and there is also the risk of a loose connection and the potential disconnection of the supply of oxygen during normal use.

Document WO 2014/124323 discloses an oxygen delivery mask comprising a port which is connectable to a mask connector, which is in turn connectable to a conduit.

There has now been devised a patient interface, a patient interface connector, a patient interface assembly, and a kit, which overcome or substantially mitigate the aforementioned and/or other disadvantages associated with the prior art.

The invention is defined by the appended claims. Subject-matter referred as embodiments, disclosures and/or inventions which are not claimed are not part of the invention. Only the embodiments comprising combinations of features defined in the claims are to be considered as embodying the invention.

According to a first aspect there is provided a patient interface assembly comprising a patient interface, a connector for connecting the patient interface to a conduit, and a conduit joined to the connector in a non-releasable manner, the patient interface comprising a first connection formation and the connector comprising a second connection formation, the patient interface and the connector being connectable such that, in a connected configuration, the first connection formation and the second connection formation are engageable with each other, with a snap fit, to provide a non-releasable connection.

According to a second aspect there is provided a kit comprising a patient interface, a separate connector for connecting the patient interface to a conduit, and a conduit joined to the connector in a non-releasable manner, wherein the patient interface comprises a first connection formation and the connector comprises a second connection formation, wherein the patient interface and the connector are connectable such that, in a connected configuration, the first connection formation and the second connection formation are engageable with each other, with a snap fit, to provide a non-releasable connection.

The first connection formation may comprise a shoulder having an abutment surface, and at least a portion of the first connection formation and/or the second connection formation may be resiliently deformable to allow the second connection formation to engage the abutment surface in the connected configuration with a snap-fit.

The patient interface assembly and kit according to the first and second aspects are advantageous principally as the patient interface and the connector are connectable with a snap-fit, which may provide a simpler and lower cost secure connection, ie non-releasable connection, relative to the prior art. The connection arrangement may also enable a simple and low cost rotatable connector. Furthermore, in a connected configuration, the first connection formation and the second connection formation cooperate to provide a non-releasable connection, which may reduce the risk of insecure connections relative to connections utilised in the prior art. In particular, the 'snap' of the snap-fit connection may inform the user that the connection is secure.

By non-releasable is meant that the connection cannot be undone by the applications of forces which are experienced during normal use of the assembly, and there is no mechanism for disengaging the connection formations from each other, and hence releasing the connection, without either breaking or fracturing of the first and/or second connection formation, or by application of a force to the patient interface and/or the connector that exceeds a pre-determined threshold force along an axis of engagement between the patient interface and the connector.

The connection may be non-releasable to the degree where the connection cannot be undone without causing structural damage to at least one of the first and second connection formations. Alternatively, the connection may be releasable only upon application of a force which exceeds a pre-determined threshold force, for example upon the application of a force which exceeds a pre-determined threshold force along an axis of engagement between the patient interface and the connector, which may or may not be a longitudinal axis of the conduit, eg away from the patient interface. The connection may be releasable only upon the application of a force, along an axis of engagement between the patient interface and the connector, having a magnitude which exceeds the forces experienced during normal use by a magnitude of at least 100%, at least 200%, at least 300%, at least 400%, at least 500%, at least 600%, at least 700% or at least 800%. The forces experienced during normal use are typically 10N or less. The pre-determined threshold force may therefore be at least 20N, at least 30N, at least 40N, at least 50N, at least 60N, at least 70N, at least 80N or at least 90N.

The first and/or second connection formation may be at least temporarily deformable upon the application of a force exceeding the pre-determined threshold, thereby disengaging the first and second connection formations and enabling release of the connection between the patient interface and the connector, without causing structural damage to, for example breaking or fracturing of, the first and/or second connection formation. The first and/or second connection formation may return to an un-deformed configuration, once the connection between the patient interface and the connector has been released, in the absence of an applied force.

This may provide a secure connection, whilst also being releasable without causing damage to the connector and/or spigot formation. Thus this connection may be safer than known non-releasable connections, where the application of forces may cause damage and/or breaking of components, which may present a choking hazard to a patient. Furthermore, the connection being releasable without causing damage to the connector and/or spigot formation may allow for reconnection of the components, if required.

According to a third aspect there is provided a patient interface assembly comprising a patient interface, a connector for connecting the patient interface to a conduit, and a conduit joined to the connector in a non-releasable manner, the patient interface comprising a first connection formation and the connector comprising a second connection formation, the first and second connection formations being engageable with each other to provide a connection between the patient interface and the connector, wherein the first and/or second connection formation is at least temporarily deformable upon the application of a force to the patient interface and/or the connector exceeding a pre-determined threshold force along an axis of engagement between the patient interface and the connector, thereby disengaging the first and second connection formations and enabling release of the connection between the patient interface and the connector, without causing structural damage to the first and/or second connection formation.

According to a fourth aspect there is provided a kit comprising a patient interface, a separate connector for connecting the patient interface to a conduit, and a conduit joined to the connector in a non-releasable manner, the patient interface comprising a first connection formation and the connector comprising a second connection formation, the first and second connection formations being engageable with each other to provide a connection between the patient interface and the connector, wherein the first and/or second connection formation is at least temporarily deformable upon the application of a force to the patient interface and/or the connector exceeding a pre-determined threshold force along an axis of engagement between the patient interface and the connector, thereby disengaging the first and second connection formations and enabling release of the connection between the patient interface and the connector, without causing structural damage to the first and/or second connection formation.

The first and/or second connection formation may return to an un-deformed configuration, once the connection between the patient interface and the connector has been released, in the absence of an applied force.

The pre-determined threshold may be at least 100%, at least 200%, at least 300%, at least 400%, at least 500%, at least 600%, at least 700% or at least 800% greater than forces experienced during normal use. The forces experienced during normal use are typically 10N or less. The pre-determined threshold may therefore be at least 20N, at least 30N, at least 40N, at least 50N, at least 60N, at least 70N, at least 80N or at least 90N.

The patient interface assembly and kit according to the third and fourth aspects are advantageous principally because the first and/or second connection formation is deformable upon the application of a force exceeding the pre-determined threshold force along an axis of engagement between the patient interface and the connector, thereby disengaging the first and second connection formations and enabling release of the connection between the patient interface and the connector, without causing structural damage to the first and/or second connection formation. In particular, these aspects may be safer than known non-releasable connections, where the application of forces may cause damage and/or breaking of components, which may present a choking hazard to a patient. Furthermore, the connection being releasable without causing damage to the connector and/or spigot formation may allow for reconnection of the components, if required.

The first and second connection formations are engageable with a snap fit.

The first connection formation may comprise a shoulder having an abutment surface, and at least a portion of the first connection formation and/or the second connection formation may be resiliently deformable to allow the second connection formation to engage the abutment surface in the connected configuration with a snap-fit.

According to a fifth aspect there is provided a packaged kit comprising packaging and a kit according to the second or fourth aspects, wherein the kit is contained within the packaging with the patient interface and the connector being in an unconnected configuration.

The kit may be entirely contained within the packaging, and may, for example be sealed within the packaging. The packaging may comprise an instruction manual, for example a manual containing instructions for use of the kit.

According to a sixth aspect there is provided a patient interface comprising a first connection formation for cooperation with a corresponding second connection formation of a connector, wherein the patient interface and the connector are connectable such that, in a connected configuration, the first connection formation and the second connection formation cooperate to provide a non-releasable connection, and wherein the first connection formation comprises a shoulder having an abutment surface for engaging the second connection formation with a snap-fit in the connected configuration.

According to a seventh aspect there is provided a patient interface connector comprising a connection formation for cooperation with a corresponding connection formation of a patient interface, wherein the patient interface and the connector are connectable such that, in a connected configuration, the corresponding connection formations cooperate to provide a non-releasable connection, and wherein at least a portion of the connector is resiliently deformable to allow the connection formation of the connector to engage an abutment surface of connection formation of the patient interface in the connected configuration with a snap-fit.

The connector may be joined to the conduit at one end thereof. The conduit may be bonded to the connector for example by adhesive bonding, solvent bonding, ultrasonic or thermal welding or the like. The conduit may be integrally formed with the connector. The conduit and the connector may be formed from a single material, for example such that the conduit and the connector form a single unitary body. The conduit and the connector may be formed as part of a single shot injection moulding process. The conduit and the connector may be formed from two or more separate materials. The connector may be overmoulded onto the conduit, for example as part of a multi-shot, insert-moulded, or co-moulded, injection moulding process.

According to an eighth aspect there is provided a patient interface assembly comprising a patient interface, a connector for connecting the patient interface to a conduit, and a conduit integrally formed with the connector as part of an injection moulding process, wherein the patient interface comprises a first connection formation and the connector comprises a second connection formation, wherein the patient interface and the connector are connectable such that, in a connected configuration, the first connection formation and the second connection formation cooperate to provide a non-releasable connection.

The patient interface assembly according to this aspect may be advantageous principally as the conduit is integrally formed with the connector as part of an injection moulding process. In particular, this may remove the need for adhesive bonding between the connector and the conduit, which may reduce the number of steps required in the manufacturing process, and/or may provide a more secure connection between the connector and the conduit. Furthermore, if any portion of the connector fractures as a result of excessive forces applied during use, then the relevant portion of the connector may remain attached to the conduit, thereby reducing the risk of fractured components of the connector forming a choking hazard.

According to a ninth aspect there is provided a kit comprising a patient interface, a separate connector for connecting the patient interface to a conduit, and a conduit integrally formed with the connector as part of an injection moulding process, wherein the patient interface comprises a first connection formation and the connector comprises a second connection formation, wherein the patient interface and the connector are connected such that, in a connected configuration, the first connection formation and the second connection formation cooperate to provide a non-releasable connection.

The conduit may be in fluid communication with the interior of the connector. The conduit may be relatively flexible, whilst the connector may be relatively rigid. The conduit may comprise polyvinylchloride (PVC), for example relatively flexible PVC. The conduit may have a Shore A hardness which is lower than the Shore A hardness of the patient interface and/or connector. The conduit may comprise a substantially constant internal diameter, for example a substantially constant circular cross-sectional area. The conduit may comprise a non-constant internal diameter, and may, for example, have a diameter which decreases from one end of the conduit to another. The internal diameter of the conduit may be tapered along its length. The conduit may comprise a conduit having ribs extending longitudinally along its internal diameter, for example such that a cross-section of the conduit comprises a substantially star-shaped internal diameter. The conduit may comprise a conduit having a so-called star lumen. The conduit may have an internal diameter in the range of 3mm to 10mm. The conduit may have an internal diameter of at most 6mm. The conduit may have a length of at least 25cm, at least 50 cm, at least 100cm, at least 200 cm, or at least 400cm.

The conduit may comprise an oxygen delivery conduit, for example a conduit which supplies oxygen to the patient interface in use.

The patient interface may comprise an oxygen delivery interface, for example an interface which provides for oxygen delivery to a patient in use. The patient interface may comprise an oxygen delivery mask, which may, for example, comprise a mask shell. The patient interface may comprise a full face mask or a nasal mask. The patient interface may comprise a sealing member, which may, for example, comprise a single walled membrane.

The mask shell may be adapted to define an internal cavity. The mask shell may be generally dome-shaped so as to define an internal cavity. The mask shell may comprise a substantially concave interior surface. The mask shell may comprise a substantially convex exterior surface.

At least a portion of the mask shell may be resiliently deformable. A region of the mask shell adjacent to a point of connection between the mask shell and the connector may be resiliently deformable. At least a portion of the mask shell adjacent to a recess or slot in the mask shell may be resiliently deformable. The mask shell may comprise polypropylene.

The mask shell may comprise at least one aperture for allowing passage of gases between the interior of the mask shell and the exterior of the mask shell. The at least one aperture may comprise an inhalation and/or exhalation aperture. The at least one aperture may be spaced apart from an oxygen inlet of the mask shell.

The patient interface may comprise a spigot extending outwardly of the patient interface. The spigot may comprise the first connection formation.

The spigot may define an inlet of the patient interface, for example an oxygen inlet. The spigot may be substantially cylindrical in form, and may comprise a hollow interior. The spigot may be substantially tubular in form, and may, for example, be open at either end. The spigot may comprise a substantially constant internal diameter, for example a substantially constant circular cross-sectional area. The spigot may comprise a non-constant internal diameter, and may, for example, have a diameter which decreases from one end of the conduit to another. The internal diameter of the spigot may be tapered along its length. The spigot may comprise a conduit having ribs extending longitudinally along its internal diameter, for example such that a cross-section of the conduit comprises a substantially star-shaped internal diameter. The spigot may comprise a conduit having a so-called star lumen. The spigot may comprise an internal diameter in the range of 3mm to 15mm. The spigot may comprise an internal diameter of at most 9mm. The spigot may comprise a length in the region of 5mm to 25mm, for example a length of around 15mm.

The spigot may comprise a female spigot, for example a spigot having a hollow interior for receiving an appropriate male projection. Alternatively, the spigot may comprise a male spigot, for example taking the form of a male projection for insertion into a corresponding hollow interior of a female spigot.

The spigot extends outwardly of the patient interface, and may, for example, extend in a direction along or orthogonal to the midsagittal plane of a user in use. The patient interface may comprise a nasal shelf. For example, the patient interface may protrude outwardly to a greater extent in a nasal region of the patient interface, such that a nasal shelf is formed. The patient interface may define the nasal shelf. For example, the nasal shelf may be defined by a wall of the mask shell of the patient interface. The mask shell of the patient interface may comprise a single unitary component. The spigot may extend outwardly from the nasal shelf, and may, for example, extend substantially orthogonally from the nasal shelf. The spigot may be integrally formed with the patient interface, for example as part of the same injection moulding process. The spigot may be integrally formed with the nasal shelf.

The patient interface may comprise an intermediate connector extending, for example orthogonally, from the nasal shelf, and the spigot may extend, for example, orthogonally, from the intermediate connector. The intermediate connector may be a separate component to the patient interface. The intermediate connector may be non-releasably connected to the patient interface.

In a preferable embodiment, the spigot may comprise the first connection formation, and the connector may comprise an enlarged head defining the second connection formation, the enlarged head being resiliently deformable to allow the second connection formation to engage the abutment surface in the connected configuration with a snap-fit.

The connector may have a substantially cylindrical global form, and may comprise a hollow interior. The connector may be substantially tubular in form, and may, for example, be open at either end. The connector may comprise a male spigot, for example taking the form of a male projection for insertion into a corresponding hollow interior of a female spigot. Alternatively, the connector may comprise a female spigot, for example a spigot having a hollow interior for receiving an appropriate male projection. The connector may comprise an external diameter corresponding substantially to the internal diameter of the spigot.

The connector may be shaped to provide a compression force to the conduit, for example to retain the connector in position relative to the conduit. The connector may comprise an internal cross-sectional shape which deforms the conduit when the connector and the conduit are connected. The connector may comprise a polygonal internal cross-sectional shape, and may, for example, comprise a polygonal internal cross-sectional shape having curved edges, for example convex curved edges. The connector may comprise a substantially constant internal diameter, and the interior of the connector may, for example comprise a substantially constant cross-sectional area and/or a substantially constant internal cross-sectional shape. The interior of the connector may comprise a plurality of different cross-sectional areas, with a transition between cross-sectional areas of a different shape. The interior of the connector may comprise a region having a circular cross-sectional area, and a region having a polygonal cross-sectional area, with a transitional region therebetween. The connector may comprise an internal diameter corresponding substantially to the external diameter of the conduit. The connector may comprise an external and/or internal diameter in the region of 3mm to 10mm. The polygonal cross-sectional area may be substantially in the shape of a reuleaux triangle.

The spigot may comprise a female spigot, and the connector may comprise a male connector, for example a male spigot, such that the connector is received within the spigot when the patient interface and the connector are connected.

Such a configuration may be beneficial as it may be easier when injection moulding, for example, to form the first connection formation on a female spigot, and to form the second connection formation on a male connector, thereby resulting in reduced manufacturing costs.

The first connection formation may comprise at least a portion of a surface, for example an internal surface, of the spigot and/or may comprise at least a portion of a surface, for example an internal surface, of the patient interface. The first and second connection formations may comprise corresponding abutment surfaces which contact each other in use. The corresponding abutment surfaces may lie substantially orthogonally to a longitudinal axis of the conduit in the connected configuration. The abutment surface of the first connection formation may comprise at least a portion of a surface, for example an internal surface, of the spigot and/or may comprise at least a portion of a surface, for example an internal surface, of the patient interface.

The first connection formation may comprise a shoulder formation, which may be located internally of the spigot and/or patient interface. For example, the first connection formation may comprise a projection which extends from a surface, for example an internal surface of the spigot and/or the patient interface, either outwardly or toward the interior of the spigot and/or the interior of the patient interface.

The second connection formation may comprise a surface, for example an external surface, of the connector. The second connection formation may comprise a surface of the connector which contacts a corresponding surface of the spigot, for example an internal surface of the spigot, in use.

The second connection formation may comprise a shoulder formation for engaging a corresponding shoulder formation of the first connection formation. The second connection formation may comprise at least one projection which extends, for example outwardly, from the connector and/or conduit, for example such that a shoulder formation is formed between the at least one projection and the connector and/or conduit. The second connection formation may comprise at least one projection which extends, for example, outwardly, from at least one arm extending from the main body of connector, for example such that a shoulder is formed between the at least one projection and the arm. The at least one projection may extend into the interior of the spigot and/or the interior of the patient interface, in use. The at least one projection may comprise an abutment surface for engaging a corresponding abutment surface of the first connection formation.

The at least one projection may extend radially outwardly from the at least one arm. The at least one projection may extend at least partially annularly about a main body of the connector. The at least one projection may be located at an end of the connector which is distal from the end of the connector that is connected to the conduit, in use. The at least one projection may be located at an end of the connector that is inserted into the patient interface first, in use.

The second connection formation may comprise a plurality of projections which extend outwardly from a plurality of arms extending from the main body of the connector. Each projection may extend from a single arm. The plurality of projections may be spaced equidistantly around the perimeter, for example the circumference, of the connector. The plurality of projections may be uniform in nature. For example, each of the plurality of projections may have substantially the same global form. The plurality of projections may be disposed in a regular pattern.

The second connection formation may be integrally formed with the connector, for example as part of a single injection moulding process. The second connection formation may comprise a region of increased diameter relative to a main body of the connector. The second connection formation may comprise an enlarged head. The diameter of the second connection formation may be greater than an internal diameter of the spigot. For example, the maximum extent between a plurality of projections of the second connection formation may be greater than an internal diameter of the spigot. The diameter of a main body of the connector may be substantially the same as an internal diameter of the spigot, for example such that an external surface of the main body of the connector is substantially in contact with an internal surface of the spigot, in use.

The at least one projection may comprise a substantially triangular cross-section, for example a cross-section taken along a central longitudinal axis of the connector. The at least one projection may comprise an angled leading edge, which may, for example, be acutely angled relative to the main body of the connector when the angle is measured between orthogonal longitudinal and transverse axes of the connector in a direction of insertion of the connector. The at least one projection may comprise an angled trailing edge, which may, for example, be obtusely angled relative to the main body of the connector when the angle is measured between orthogonal longitudinal and transverse axes of the connector in a direction of insertion of the connector. This may allow the non-releasable connection to be broken upon the application of a force which exceeds the forces experienced during normal use. The trailing edge may be angled at an angle in the range of 90° to 120°. The trailing edge may be angled at at most 91°, at most 92°, at most 93°, at most 94°, at most 95°, or at most 100°. The at least one projection may define a holding barb at an end of the connector, for example the end of the connector which is inserted first into the spigot, in use.

At least a portion of the connector may be resiliently deformable, for example such that at least a portion of the connector deforms in response to an applied pressure, but substantially regains its original shape when the applied pressure is removed. The second connection formation and/or the at least one projection and/or the at least one arm, may be resiliently deformable.

The connector may comprise at least one slot which enables at least a portion of the connector to resiliently deform relative to the remainder of the connector. The at least one slot may enable the second connection formation to resiliently deform relative to the remainder of the connector. The at least one slot may extend longitudinally along the connector, for example along the main body of the connector. The at least one slot may extend parallel to the at least one arm.

The at least one slot may enable the second connection formation to resiliently deform relative to the remainder of the connector upon insertion of the connector into the spigot. The second connection formation may be shaped and/or dimensioned such that the second connection formation does not resiliently deform upon attempted removal of the connector from the spigot, at least under loads which are applied during normal operation. The second connection formation may resiliently deform upon attempted removal of the connector from the spigot where the force applied is greater than forces which are typically experienced during normal operating conditions. The second connection may resiliently deform upon application of a force greater than a pre-determined threshold force. The pre-determined threshold force may be at least 100%, at least 200%, at least 300%, at least 400%, at least 500%, at least 600%, at least 700%, or at least 800% greater than forces experiences during normal use. The pre-determined threshold may therefore be at least 20N, at least 30N, at least 40N, at least 50N, at least 60N, at least 70N, at least 80N or at least 90N.

The connector may comprise a plurality of slots, each of which extends longitudinally along the connector. The plurality of slots may be substantially uniform in nature, for example such that each of the plurality of slots has substantially the same global form. The plurality of slots may be disposed evenly around the circumference of the connector, for example in a regular pattern. Each of the plurality of slots may be located adjacent to a projection and/or may be located intermediate adjacent projections. Each of the plurality of slots may extend alongside an arm.

The first connection formation may comprise a recess for receiving at least a portion of the second connection formation. For example, the first connection formation may comprise a slot formed in an outer surface of the patient interface. Such a configuration may be beneficial as this may reduce the complexity of the manufacturing process of the patient interface. For example, such a configuration may remove the need for moving cores in the tool for moulding the patient interface. The tool may be a so-called "open and shut" tool, and may mean that more cavities can be included in the tool to make the patient interface. This may reduce the cycle time for the moulding of the patient interface, and may allow for more shots per cycle.

The recess may be configured to receive the second connection formation in a direction which is obliquely angled relative to, or substantially orthogonal to, a longitudinal axis of the conduit. An axis of insertion of the connector into the recess may be obliquely angled relative to, or substantially orthogonal to, a longitudinal axis of the conduit. An axis of insertion of the second connection formation may be obliquely angled relative to, or substantially orthogonal to, a longitudinal axis of the conduit.

The recess may extend in a direction which is angled relative to a longitudinal axis of the conduit when the patient interface and the connector are in a connected configuration. The recess may extend in a direction which is obliquely angled relative to, or substantially orthogonal to, a longitudinal axis of the conduit when the patient interface and the connector are in a connected configuration. The recess may extend in a direction which requires insertion of the second connection formation into the recess in a direction which is substantially orthogonal to a longitudinal axis of the conduit. The recess may extend along a direction of a nasal shelf of the patient interface. The recess may, for example, extend into the nasal shelf, such that at least a portion of the nasal shelf defines a nasal shelf aperture for receiving at least a portion of the connector in a connected configuration. The nasal shelf aperture may have a width, for example a diameter, that is less than a corresponding width of the recess of the first connection formation.

The recess may be defined by at least one internal wall of the patient interface and/or a nasal shelf wall of the patient interface. The at least one internal wall of the patient interface may prevent removal of the connector from the recess in a direction substantially orthogonal to a longitudinal axis of the conduit in the connected configuration. The nasal shelf wall of the patient interface may prevent removal of the connector from the recess in a direction substantially parallel to a longitudinal axis of the conduit in the connected configuration.

In a preferable embodiment of the present invention, the recess may comprise the first connection formation, and the connector may comprise an enlarged head defining the second connection formation, and at least a portion of the first connection formation may be resiliently deformable to allow the second connection formation to engage the abutment surface in the connected configuration with a snap-fit.

The shoulder of the first connection formation may be located internally of the patient interface, and may for example, be formed on wall which at least partially defines the recess of the first connection formation. The shoulder may comprise an annular shoulder. Substantially the entirety of the perimeter of the annular shoulder may engage a corresponding portion of the second connection formation in a connected configuration.

At least a portion of the patient interface may resiliently deform to allow insertion of the connector into the recess, and may resiliently deform into its original shape once the connector has been fully inserted into the recess. The shoulder may prevent removal of the connector from the recess in a direction substantially orthogonal to a longitudinal axis of the conduit in the connected configuration.

The first and second connection formations may comprise a plurality of corresponding pairs abutment surfaces which contact each other in use. A first pair of corresponding abutment surfaces may lie substantially orthogonally to a longitudinal axis of the conduit in the connected configuration, whilst a second pair of corresponding abutment surfaces may lie substantially parallel to a longitudinal axis of the conduit in the connected configuration. Thus the first and second connection formations may resist disconnection in more than one direction, when in the connected configuration.

The second connection formation may comprise a surface, for example an external surface, of the connector. The second connection formation may comprise a surface of the connector which contacts a corresponding surface of the patient interface, for example an internal surface of the patient interface, in use.

The second connection formation may comprise a shoulder formation for engaging a corresponding shoulder formation of the first connection formation. The second connection formation may comprise at least one projection which extends, for example outwardly, from the connector and/or conduit, for example such that a shoulder formation is formed between the at least one projection and the connector and/or conduit. The at least one projection may extend into the interior of the recess of the patient interface and/or the interior of the patient interface, in use. The at least one projection may comprise an abutment surface for engaging a corresponding abutment surface of the first connection formation.

The at least one projection may extend radially outwardly from a main body of the connector. The at least one projection may extend at least partially annularly about the main body of the connector. The at least one projection may be upstanding from an end of the connector, for example an end of the connector which is distal from the end of the connector that is connected to the conduit, in use. The at least one projection may extend from a main body of the connector in a direction which is substantially parallel to a longitudinal axis of the connector.

The at least one projection may be located at an end of the connector which is distal from the end of the connector that is connected to the conduit, in use. The at least one projection may be located at an end of the connector that is inserted into the patient interface first, in use.

The second connection formation may comprise a plurality of projections which extend outwardly from the main body of the connector. Each projection may extend in substantially orthogonal directions. A first projection may extend substantially radially outwardly from a main body of the connector, whilst a second projection may be upstanding from an end of the connector, in a direction along a longitudinal axis of the connector.

The second connection formation may be integrally formed with the connector, for example as part of a single injection moulding process. The second connection formation may comprise a region of increased diameter relative to a main body of the connector. The second connection formation may comprise an enlarged head. The diameter of a main body of the connector may be substantially the same as an internal diameter of the recess in the patient interface, for example such that an external surface of the main body of the connector is substantially in contact with an internal surface of the recess in the patient interface, in use.

The at least one projection may comprise a substantially triangular cross-section, for example a cross-section taken along a central longitudinal axis of the connector. The at least one projection may comprise an angled leading edge, which may, for example, be acutely angled relative to the main body of the connector when the angle is measured between orthogonal longitudinal and transverse axes of the connector in a direction orthogonal to that of insertion of the connector. The at least one projection may define a holding barb at an end of the connector, for example the end of the connector which is inserted first into the recess in the patient interface, in use.

The connector may be rotatably connected to the patient interface in use, for example such that the connector is rotatable relative to the patient interface, and vice versa, in use. The first and second connection formations may be rotatably connected, in use. The abutment surfaces of the first and second connection formations may enable relative rotational movement therebetween. The connector may be rotatable about a longitudinal axis of the connector, for example a central longitudinal axis of the connector, in use.

The patient interface and the connector may be connected by a snap-fit connection, in use. The first and second connection formations may be connected by a snap-fit, in use. For example, the second connection formation may deform upon insertion of the connector into the spigot, and the second connection formation may regain its original shape once it has passed through the internal diameter of the spigot, such that the first and second connection formations are in engagement.

The first and/or second connection formation may be configured to resist resilient deformation upon attempted removal of the connector from the patient interface, such that the connector cannot be removed from the patient interface, or vice versa, unless the removal force applied is greater than forces which are experienced during normal operating conditions, for example unless the removal force is greater than a pre-determined threshold force. The pre-determined threshold force may be at least 100%, at least 200%, at least 300%, at least 400%, at least 500%, at least 600%, at least 700%, or at least 800% greater than forces experiences during normal use. The pre-determined threshold may therefore be at least 20N, at least 30N, at least 40N, at least 50N, at least 60N, at least 70N, at least 80N or at least 90N. Alternatively, the first and/or second connection formation may be configured to resist resilient deformation upon attempted removal of the connector from the patient interface, such that the connector cannot be removed from the patient interface without causing structural breakage of the connector and/or patient interface. The first and second connection formations may be in engagement, and may be disposed substantially orthogonally relative to a direction of attempted removal of the connector from the patient interface, in the connected configuration, such that the connector cannot be removed from the patient interface, or vice versa, without causing damage to the patient interface and/or the connector. The first and/or second connection formations may form an acute and/or perpendicular angle relative to the main body of the connector in the connected configuration, the angle being measured between the main body of the connector and an axis of insertion of the connector, in a direction of insertion of the connector.

The spigot may comprise a single wall construction, for example such that the spigot is defined by a single wall. The spigot may be integrally formed with the patient interface, for example as part of a single injection moulding process.

The spigot and/or patient interface and/or connector may comprise a stop formation for preventing over insertion of the connector into the patient interface in use. The stop formation(s) may inhibit movement of the connector relative to the patient interface, along a longitudinal axis of the connector, in use. The spigot and/or patient interface may comprise a first stop formation, and the connector may comprise a second stop formation, such that the first and second stop formations cooperate, in use, to prevent over insertion of the connector into the patient interface. The first and/or second stop formations may be integrally formed with their respective components, for example as part of a single injection moulding process.

The first and/or second stop formations may comprise corresponding abutment surfaces, which may, for example, engage one another in use. The first stop formation may comprise an internal and/or external surface of the spigot, for example a surface located at an end of the spigot. The second stop formation may comprise an internal and/or external surface of the connector. The corresponding abutment surfaces may lie substantially orthogonally to a longitudinal axis of the conduit in the connected configuration.

The first and/or second stop formations may comprise corresponding projections and/or recesses, for example corresponding annular projections and/or recesses. The first stop formation may comprise an annular recess formed on an internal surface of the spigot, and the second stop formation may comprise an annular projection formed on an external surface of the connector. The second stop formation may fit substantially within the first stop formation, in use. The second stop formation and the second connection formation may be located at opposing ends of the connector. The first stop formation may comprise an end surface of the spigot, and the second stop formation may comprise an annular projection formed on an external surface of the connector. The second stop formation may abut substantially the entirety of the end surface of the spigot, in use.

The patient interface may comprise a rigid patient interface, for example a patient interface with a relatively low degree of flexibility which maintains its shape when subjected to normal handling conditions. The patient interface may have a Shore A hardness greater than the Shore A hardness of the conduit. The patient interface may be formed from plastics material, for example using an injection moulding process. The patient interface may comprise polypropylene.

The connector may comprise a rigid connector, for example a connector with a relatively low degree of flexibility which maintains its shape when subjected to normal handling conditions. The connector may have a Shore A hardness greater than the Shore A hardness of the conduit. The connector may be formed from plastics material, for example using an injection moulding process. The connector may comprise polyvinylchloride (PVC), for example rigid PVC.

Practicable embodiments will now be described with reference to the accompanying drawings, of which:
Figure 1 is a perspective view of a patient interface assembly according to a first embodiment of the invention;
Figure 2 is an enlarged cross-sectional view of the connection interface of the patient interface assembly of Figure 1;
Figure 3 is a perspective view of the connector utilised in Figures 1 and 2;
Figure 4 is a cross-sectional view of a first alternative embodiment of a connector for use in the first embodiment of the patient interface assembly of the present invention
Figure 5 is a perspective view of a second alternative embodiment of a connector for use in the first embodiment of the patient interface assembly of the present invention;
Figure 6 is a perspective view of a third alternative embodiment of a connector for use in the first embodiment of the patient interface assembly of the present invention;
Figure 7 is a cross-sectional view of a fourth alternative embodiment of a connector for use in the first embodiment of the patient interface assembly of the present invention;
Figure 8 is an enlarged perspective view of a patient interface assembly according to a second embodiment of the invention;
Figure 9 is a first enlarged cross-sectional view of the connection interface of the patient interface assembly of Figure 8; and
Figure 10 is a first enlarged cross-sectional view of the connection interface of the patient interface assembly of Figure 8.

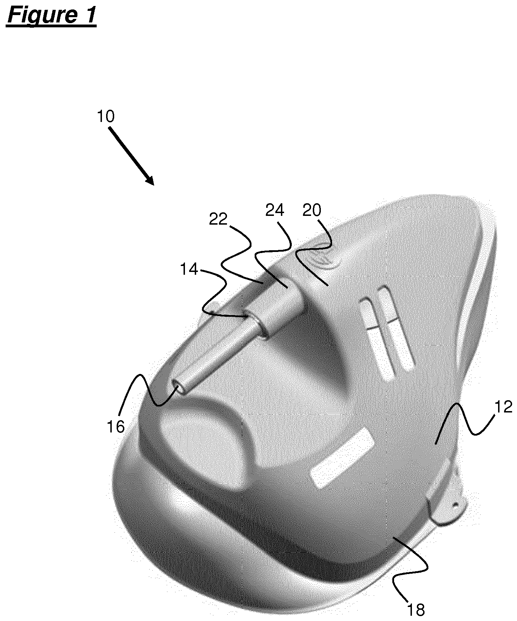

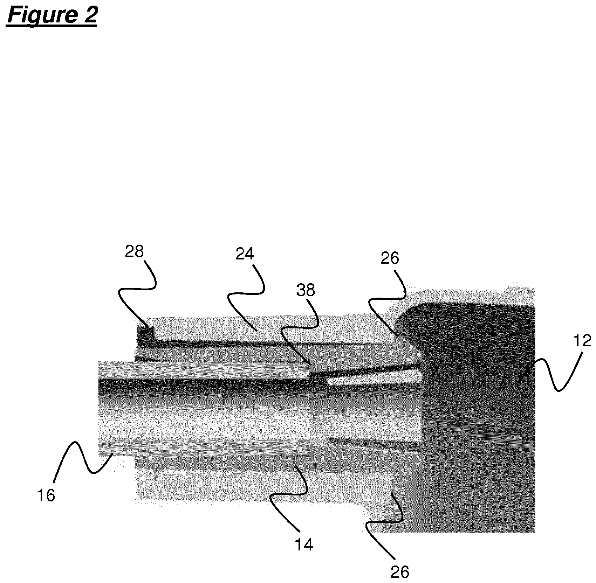

A patient interface assembly according to a first embodiment of the present invention, generally designated 10, is shown in Figures 1 and 2.

The patient interface assembly 10 comprises an oxygen delivery mask 12, a connector 14, and an oxygen conduit 16.

The oxygen delivery mask 12 comprises a rigid mask body 18, formed of polypropylene. The mask body 18 is generally concave in from, and defines an internal cavity. A nasal region 20 of the mask body 18 extends forwardly of the remainder of the mask body 18, thereby defining a shelf 22. A female spigot 24 depends from the shelf 22.

The female spigot 24 is substantially cylindrical in global form, and has a hollow interior. The internal diameter of the female spigot 24 is substantially the same is the external diameter of the main body of the connector 14. A first end of the female spigot 24 defines an internal annular shoulder 26, whilst a second end of the female spigot 24 defines an internal annular recess 28.

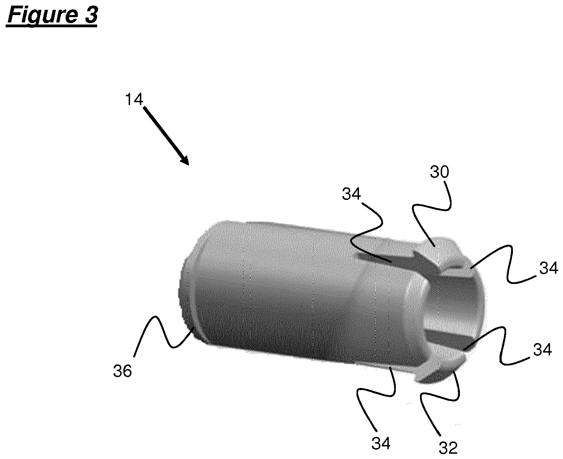

The connector 14 is shown in more detail in Figures 2 and 3. The connector 14 has a substantially cylindrical global form, and is hollow. The connector 14 is generally rigid in nature, and is formed of polyvinylchloride (PVC). A first end of the connector 14 comprises first 30 and second 32 projections, and a plurality of slots 34. The first 30 and second 32 projections are integrally formed with, and extend radially outwardly from, the main body of the connector 14. The first 30 and second 32 projections thereby form a region of increased diameter relative to the main body of the connector 14. Each of the plurality of slots 34 is located adjacent a corresponding projection 30,32, and extends longitudinally along the connector 14.

A second end of the connector 14 has an annular projection 36. The annular projection 36 is integrally formed with the connector 14, and is shaped and dimensioned to engage the corresponding internal annular recess 28 of the female spigot 24 in use. The connector 14 further comprises an internal abutment shoulder 38 for preventing over-insertion of a conduit 16 into the connector 14.

In use, a conduit 16 is bonded to the interior of the connector 14 using adhesive glue, such that a flow path is defined through the interior of the conduit 14. The connector 14 is inserted into the female spigot 24. The slots 34 allow the region of the connector 14 having the first 30 and second 32 projections to deform during passage of the connector 14 through the female spigot 24. Once the first 30 and second 32 projections have passed through the interior of the female spigot 24 and into the interior of the mask body 18, the first 30 and second 32 projections resiliently deform such that the connector 14 reverts to its original pre-deformation shape. In other words, the connector 14 is connected to the oxygen delivery mask 12 with a snap-fit connection

The first 30 and second 32 projections are now in contact with the internal annular shoulder 26 of the female spigot 24, whilst the annular projection 36 of the connector 14 is in contact with the internal annular recess 28 of the female spigot 24. Movement of the connector 14 along a longitudinal axis of the connector 14 is now inhibited by the engagement between the first 30 and second 32 projections and the internal annular shoulder 26, as well as by the engagement between the annular projection 36 and the internal annular recess 28. Thus the connector 14 cannot be removed from the oxygen delivery mask 12 without causing damage to either the connector 14 or the oxygen delivery mask 12. The nature of the connection still enables the connector 14 to rotate about its central longitudinal axis, which inhibits the formation of any kinks or strain in the conduit 16.

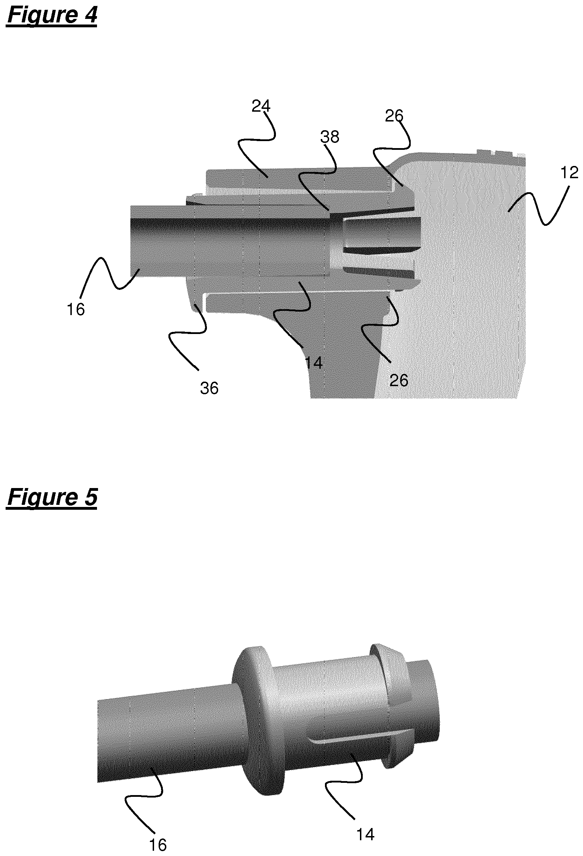

A first alternative embodiment of a connector 14 is shown in Figure 4. The embodiment of the connector 14 shown in Figure 4 is substantially the same as the embodiment of the connector 14 shown in Figures 2 and 3, and differs only in that the annular projection 36 extends outwardly from the body of the connector 14 to a much greater degree, such that the annular projection abuts the end surface of the spigot 24 in use. This prevents over insertion into the spigot 24, and removes the need for the internal annular shoulder 26 of the spigot 24.

A second alternative embodiment of a connector 14 is shown in Figure 5. The connector of Figure 5 is substantially the same as the connector of Figure 4, and differs only in that the connector 14 is overmoulded directly onto the conduit 16.

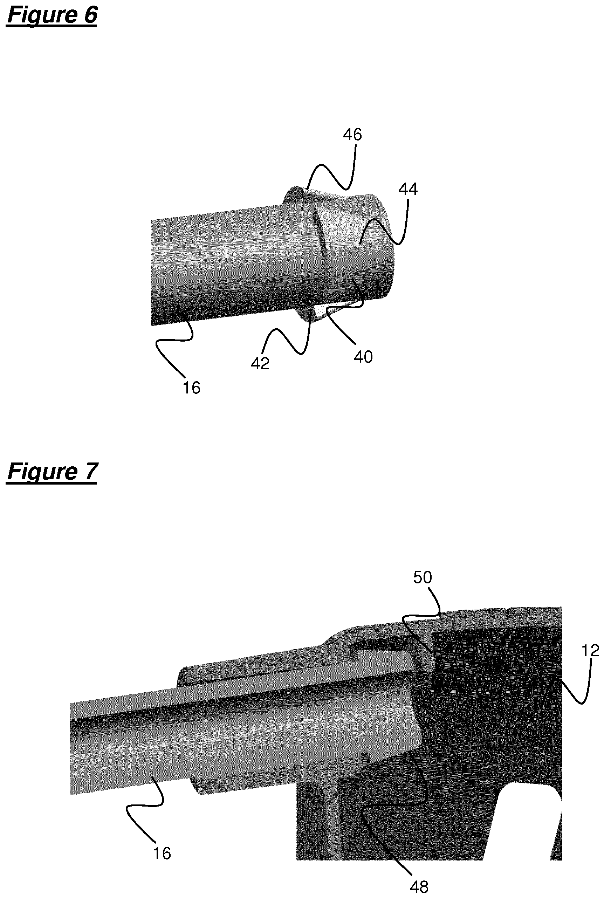

A third alternative embodiment of a connector 40 is shown in Figure 6. As in the embodiment of Figure 5, the connector 40 is over-moulded directly onto the conduit 16. The connector 40 comprises a first 42, second 44, and third 46, projections spaced circumferentially about the conduit 16. The projections 42,44,46 have a form similar to the projections 30,32 of the previously discussed connectors 14, and operate in a similar manner to retain the connector 40 relative to the oxygen delivery mask 12.

A fourth alternative embodiment of a connector 48 is shown in Figure 7, attached to an oxygen delivery mask 12. The connector 48 is integrally formed with the conduit 16 as part of a single injection moulding process, and takes the form of an annular projection extending about one end of the conduit 16 to form an enlarged head. The connector 48 operates in a similar manner to other embodiments of connectors 14, 40 previously discussed. In use, the connector 48 abuts an internal wall 50 of the oxygen delivery mask 12, to prevent over insertion of the conduit 16 into the mask 12.

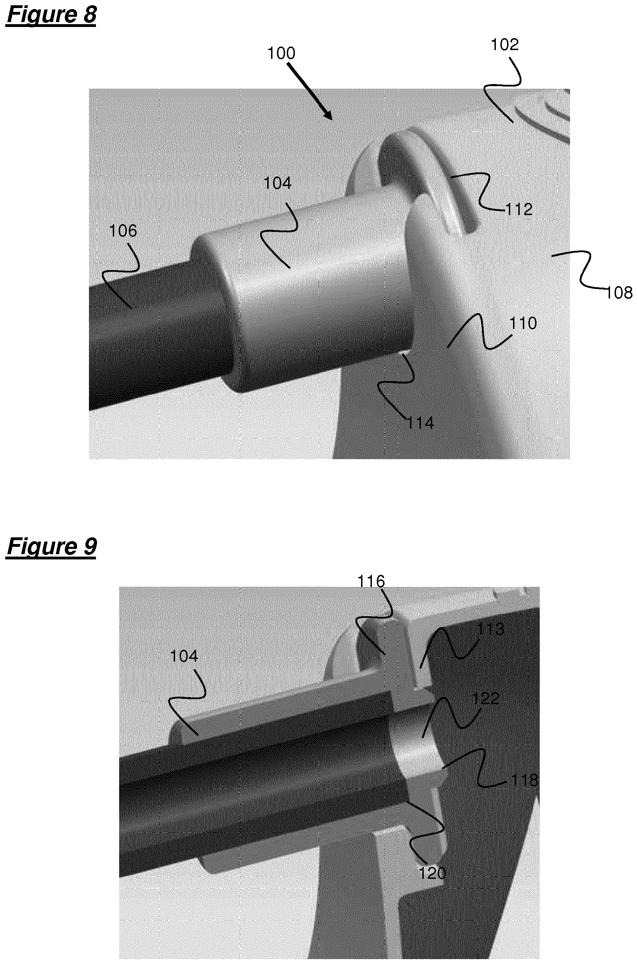

A second embodiment of a patient interface assembly according to the present invention, generally designated 100, is shown in Figures 8 to 10.

The patient interface assembly 100 comprises an oxygen delivery mask 102, a connector 104, and an oxygen conduit 106.

The oxygen delivery mask 102 comprises a rigid mask body 108, formed of polypropylene. The mask body 108 is generally concave in from, and defines an internal cavity. A nasal region of the mask body 108 extends forwardly of the remainder of the mask body 108, thereby defining a shelf 110. A slot 112 is formed in the mask body 108 slightly above the shelf 110, whilst an aperture 114 is formed in the shelf 110 itself. The slot 112 has a diameter larger than the diameter of the aperture 114. The aperture 114 has a diameter corresponding substantially to the diameter of the main body of the conduit 104. The slot 112 is formed between an internal wall 113 of the mask body 108 and the shelf 110. The internal wall 113 defines a recess 115 which receives a second projection 118 of the conduit 104 in a connected configuration

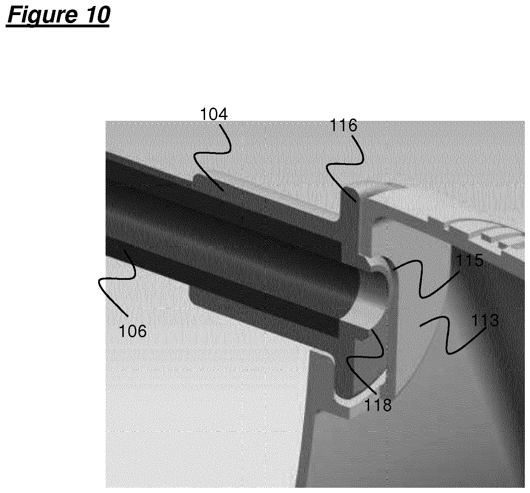

The connector 104 is shown in more detail in Figures 9 and 10. The connector 104 has a substantially cylindrical global form, and is hollow. The connector 104 is generally rigid in nature, and is formed of polyvinylchloride (PVC). A first end of the connector 104 comprises first 116 and second 118 projections. The first 116 and second 118 projections are integrally formed with the connector. The connector 104 further comprises an internal abutment shoulder 120 for preventing over-insertion of a conduit 106 into the connector 104.

The first projection 116 extends annularly about an end the main body of the connector 104, such that the first projection 116 forms an enlarged head of the connector 104 at one end thereof. The diameter of the first projection 116 is substantially the same as that of the slot 112. The second projection 118 is upstanding from the same end of the connector 104, and is substantially annular in form. The second projection 118 defines an aperture 122 which is shaped and dimensioned to correspond to the internal diameter of the conduit 106. The outer surface of the second projection 118 is shaped to define an angular upper portion, and a linear lower portion, as seen in Figure 10. The second projection 118 is resiliently deformable.

In use, a conduit 106 is bonded to the interior of the connector 104 using adhesive glue, such that a flow path is defined through the interior of the conduit 104. The connector 104 is inserted into the slot 112 via the first projection 116. When the angled upper outer surface of the second projection 118 first engages the mask body 108, the mask body 108 in the region of the slot 112 resiliently deforms to allow continued insertion of the connector 104 into the slot 112. Upon further insertion of the connector 104, the second projection 118 engages the recess 115 in the internal wall 113, and the mask body 108 returns to its original shape, such that the connector 104 engages the mask body 108 with a snap-fit. In particular, the linear lower portion of the outer surface of the second projection 118 engages the internal wall 113. The main body of the connector 104 is received within the aperture 114.

In the connected configuration, the linear lower portion of the outer surface of the second projection 118 engages the internal wall 113, to prevent removal of the connector 104 from the mask body 108 in a direction that is orthogonal to a longitudinal axis of the conduit 106. The second projection 116 engages the shelf 110 to prevent removal of the connector 104 from the mask body 108 in a direction that is parallel to a longitudinal axis of the conduit 106.

## Claims

1. An oxygen delivery interface assembly (10, 100) comprising a patient interface (12, 102), a connector (14, 104) for connecting the patient interface to a conduit (16, 106), and a conduit joined to the connector in a non-releasable manner, the patient interface comprising a first connection formation and the connector comprising a second connection formation, the second connection formation comprising at least one projection (30, 32, 42, 44, 46, 118), the patient interface and the connector being connectable such that, in a connected configuration, the first connection formation and the second connection formation are engageable with each other, with a snap fit, to provide a non-releasable connection in which the at least one projection extends from the connector into the interior of the patient interface.

2. An oxygen delivery interface assembly as claimed in Claim 1, wherein the connection is non-releasable to the degree where the connection cannot be undone without causing structural damage to at least one of the first and second connection formations.

3. An oxygen delivery interface assembly as claimed in Claim 1, wherein the connection is releasable only upon application of a force that exceeds a pre-determined threshold force along an axis of engagement between the patient interface and the connector.

4. An oxygen delivery interface assembly as claimed in Claim 3, wherein the connection is releasable only upon the application of a force, along an axis of engagement between the patient interface and the connector, having a magnitude at least 20N, at least 30N, at least 40N, at least 50N, at least 60N, at least 70N, at least 80N or at least 90N.

5. An oxygen delivery interface assembly as claimed Claim 3 or Claim 4, wherein the first and/or second connection formation is at least temporarily deformable upon the application of a force exceeding the pre-determined threshold, thereby disengaging the first and second connection formations and enabling release of the connection between the patient interface and the connector, without causing structural damage to, for example breaking or fracturing of, the first and/or second connection formation.

6. An oxygen delivery interface assembly as claimed in any preceding claim, wherein the first connection formation comprises a recess (115) for receiving at least a portion of the second connection formation.

7. An oxygen delivery interface assembly as claimed in Claim 6, wherein the first connection formation comprises a slot (112) formed in an outer surface of the patient interface, for receiving at least a portion of the second connection formation.

8. An oxygen delivery interface assembly as claimed in Claim 6 or Claim 7, wherein the recess extends in a direction which requires insertion of the second connection formation into the recess in a direction which is substantially orthogonal to a longitudinal axis of the conduit.

9. An oxygen delivery interface assembly as claimed in any of Claims 6 to 8, wherein the recess is defined by at least one internal wall of the patient interface and a nasal shelf wall of the patient interface.

10. An oxygen delivery assembly as claimed in Claim 9, wherein the at least one internal wall of the patient interface prevents removal of the connector from the recess in a direction orthogonal to a longitudinal axis of the conduit in the connected configuration, and wherein the nasal shelf wall of the patient interface prevents removal of the connector from the recess in a direction substantially parallel to a longitudinal axis of the conduit in the connected configuration.

11. An oxygen delivery interface assembly as claimed in any preceding claim, wherein the first connection formation comprises at least a portion of an internal surface of the patient interface.

12. An oxygen delivery interface assembly as claimed in any preceding claim, wherein the second connection formation comprises a shoulder for engaging a corresponding shoulder of the first connection formation.

13. An oxygen delivery interface assembly as claimed in any preceding claim, wherein the second connection formation comprises a region of increased diameter relative to a main body of the connector.

14. An oxygen delivery interface assembly as claimed in Claim 13, wherein the second connection formation extends at least partially annularly about the main body of the connector.

15. An oxygen delivery interface assembly as claimed in any preceding claim, wherein the first and second connection formations are rotatably connected, in use.

16. An oxygen delivery interface assembly as claimed in any preceding claim, wherein the patient interface comprises a first stop formation, and the connector comprises a second stop formation, such that the first and second stop formations cooperate, in use, to prevent over insertion of the connector into the patient interface, and wherein the first and/or second stop formation comprise annular projections and/or recesses.

## Patentansprüche

1. Schnittstellenanordnung (10, 100) zur Sauerstoffzufuhr, umfassend eine Patientenschnittstelle (12, 102), ein Anschlussstück (14, 104) zum Verbinden der Patientenschnittstelle mit einer Leitung (16, 106) und eine Leitung, die mit dem Anschlussstück in einer nicht lösbaren Weise verbunden ist, wobei die Patientenschnittstelle eine erste Verbindungsausbildung umfasst und das Anschlussstück eine zweite Verbindungsausbildung umfasst, wobei die zweite Verbindungsausbildung mindestens einen Vorsprung (30, 32, 42, 44, 46, 118) umfasst, wobei die Patientenschnittstelle und das Anschlussstück derart verbindbar sind, dass in einer verbundenen Konfiguration die erste Verbindungsausbildung und die zweite Verbindungsausbildung mit einem Schnappsitz miteinander in Eingriff bringbar sind, um eine nicht lösbare Verbindung bereitzustellen, in der sich der mindestens eine Vorsprung von dem Anschlussstück in das Innere der Patientenschnittstelle erstreckt.

2. Schnittstellenanordnung zur Sauerstoffzufuhr nach Anspruch 1, wobei die Verbindung in dem Maße nicht lösbar ist, in dem die Verbindung nicht gelöst werden kann, ohne eine strukturelle Beschädigung an mindestens einer von der ersten und zweiten Verbindungsausbildung zu verursachen.

3. Schnittstellenanordnung zur Sauerstoffzufuhr nach Anspruch 1, wobei die Verbindung nur bei Aufbringen einer Kraft lösbar ist, die eine vorbestimmte Schwellenkraft entlang einer Eingriffsachse zwischen der Patientenschnittstelle und dem Anschlussstück überschreitet.

4. Schnittstellenanordnung zur Sauerstoffzufuhr nach Anspruch 3, wobei die Verbindung nur bei Anwendung einer Kraft entlang einer Eingriffsachse zwischen der Patientenschnittstelle und dem Anschlussstück mit einer Größe von mindestens 20 N, mindestens 30 N, mindestens 40 N, mindestens 50 N, mindestens 60 N, mindestens 70 N, mindestens 80 N oder mindestens 90 N lösbar ist.

5. Schnittstellenanordnung zur Sauerstoffzufuhr nach Anspruch 3 oder Anspruch 4, wobei die erste und/oder zweite Verbindungsausbildung bei Anwendung einer Kraft, die den vorbestimmten Schwellenwert überschreitet, zumindest zeitweise verformbar ist, wodurch die erste und zweite Verbindungsausbildung gelöst werden und das Lösen der Verbindung zwischen der Patientenschnittstelle und dem Anschlussstück ermöglicht wird, ohne eine strukturelle Beschädigung, beispielsweise ein Brechen oder Zerbrechen, an der ersten und/oder zweiten Verbindungsausbildung zu verursachen.

6. Schnittstellenanordnung zur Sauerstoffzufuhr nach einem der vorhergehenden Ansprüche, wobei die erste Verbindungsausbildung eine Aussparung (115) zum Aufnehmen mindestens eines Teils der zweiten Verbindungsausbildung umfasst.

7. Schnittstellenanordnung zur Sauerstoffzufuhr nach Anspruch 6, wobei die erste Verbindungsausbildung einen Schlitz (112) umfasst, der in einer Außenfläche der Patientenschnittstelle ausgebildet ist, um mindestens einen Teil der zweiten Verbindungsausbildung aufzunehmen.

8. Schnittstellenanordnung zur Sauerstoffzufuhr nach Anspruch 6 oder Anspruch 7, wobei sich die Aussparung in eine Richtung erstreckt, die das Einführen der zweiten Verbindungsausbildung in die Aussparung in einer Richtung erfordert, die im Wesentlichen orthogonal zu einer Längsachse der Leitung ist.

9. Schnittstellenanordnung zur Sauerstoffzufuhr nach einem der Ansprüche 6 bis 8, wobei die Aussparung durch mindestens eine Innenwand der Patientenschnittstelle und eine Nasenablagewand der Patientenschnittstelle definiert ist.

10. Sauerstoffzufuhranordnung nach Anspruch 9, wobei die mindestens eine Innenwand der Patientenschnittstelle das Entfernen des Anschlussstücks aus der Aussparung in einer Richtung orthogonal zu einer Längsachse der Leitung in der verbundenen Konfiguration verhindert, und wobei die Nasenablagewand der Patientenschnittstelle das Entfernen des Anschlussstücks aus der Aussparung in einer Richtung im Wesentlichen parallel zu einer Längsachse der Leitung in der verbundenen Konfiguration verhindert.

11. Schnittstellenanordnung zur Sauerstoffzufuhr nach einem der vorhergehenden Ansprüche, wobei die erste Verbindungsausbildung mindestens einen Teil einer Innenfläche der Patientenschnittstelle umfasst.

12. Schnittstellenanordnung zur Sauerstoffzufuhr nach einem der vorhergehenden Ansprüche, wobei die zweite Verbindungsausbildung eine Schulter zum Eingriff in eine entsprechende Schulter der ersten Verbindungsausbildung umfasst.

13. Schnittstellenanordnung zur Sauerstoffzufuhr nach einem der vorhergehenden Ansprüche, wobei die zweite Verbindungsausbildung einen Bereich mit vergrößertem Durchmesser relativ zum Hauptkörper des Anschlussstücks umfasst.

14. Schnittstellenanordnung zur Sauerstoffzufuhr nach Anspruch 13, wobei sich die zweite Verbindungsausbildung zumindest teilweise ringförmig um den Hauptkörper des Anschlussstücks erstreckt.

15. Schnittstellenanordnung zur Sauerstoffzufuhr nach einem der vorhergehenden Ansprüche, wobei die erste und zweite Verbindungsausbildung im Gebrauch drehbar verbunden sind.

16. Schnittstellenanordnung zur Sauerstoffzufuhr nach einem der vorhergehenden Ansprüche, wobei die Patientenschnittstelle eine erste Anschlagausbildung umfasst und das Anschlussstück eine zweite Anschlagausbildung umfasst, sodass die erste und zweite Anschlagausbildung im Gebrauch zusammenwirken, um ein übermäßiges Einführen des Anschlussstücks in die Patientenschnittstelle zu verhindern, und wobei die erste und/oder zweite Anschlagausbildung ringförmige Vorsprünge und/oder Aussparungen umfassen.

## Revendications

1. Ensemble interface d'administration d'oxygène (10, 100) comprenant une interface patient (12, 102), un connecteur (14, 104) destiné à raccorder l'interface patient à un conduit (16, 106) et un conduit relié au connecteur de manière non libérable, l'interface patient comprenant une première formation de raccord et le connecteur comprenant une seconde formation de raccord, la seconde formation de raccord comprenant au moins une saillie (30, 32, 42, 44, 46, 118), l'interface patient et le connecteur pouvant être raccordés de sorte que, dans une configuration raccordée, la première formation de raccord et la seconde formation de raccord puissent se mettre en prise l'une avec l'autre, par encliquetage, pour fournir un raccord non libérable dans laquelle la au moins une saillie s'étend à partir du connecteur jusque dans l'intérieur de l'interface patient.

2. Ensemble interface d'administration d'oxygène selon la revendication 1, ledit raccord étant non libérable au point où le raccord ne peut pas être détaché sans causer de dommages structurels à au moins l'une des première et seconde formations de raccord.

3. Ensemble interface d'administration d'oxygène selon la revendication 1, ledit raccord étant libérable uniquement lors de l'application d'une force qui dépasse une force seuil prédéfinie le long d'un axe de mise en prise entre l'interface patient et le connecteur.

4. Ensemble interface d'administration d'oxygène selon la revendication 3, ledit raccord étant libérable uniquement lors de l'application d'une force, le long d'un axe de mise en prise entre l'interface patient et le connecteur, possédant une amplitude supérieure ou égale à 20 N, supérieure ou égale à 30 N, supérieure ou égale à 40 N, supérieure ou égale à 50 N, supérieure ou égale à 60 N, supérieure ou égale à 70 N, supérieure ou égale à 80 N ou supérieure ou égale à 90 N.

5. Ensemble interface d'administration d'oxygène selon la revendication 3 ou la revendication 4, ladite première et/ou ladite seconde formation de raccord étant au moins temporairement déformable lors de l'application d'une force dépassant le seuil prédéfini, séparant ainsi les première et seconde formations de raccord et permettant la libération du raccord entre l'interface patient et le connecteur, sans causer de dommages structurels, par exemple une rupture ou une fracturation, de la première et/ou de la seconde formation de raccord.

6. Ensemble interface d'administration d'oxygène selon une quelconque revendication précédente, ladite première formation de raccord comprenant un évidement (115) destiné à recevoir au moins une partie de la seconde formation de raccord.

7. Ensemble interface d'administration d'oxygène selon la revendication 6, ladite première formation de raccord comprenant une fente (112) formée dans une surface externe de l'interface patient, destinée à recevoir au moins une partie de la seconde formation de raccord.

8. Ensemble interface d'administration d'oxygène selon la revendication 6 ou la revendication 7, ledit évidement s'étendant dans une direction qui nécessite l'insertion de la seconde formation de raccord dans l'évidement dans une direction qui est sensiblement orthogonale à un axe longitudinal du conduit.

9. Ensemble interface d'administration d'oxygène selon l'une quelconque des revendications 6 à 8, ledit évidement étant défini par au moins une paroi interne de l'interface patient et une paroi d'étage nasale de l'interface patient.

10. Ensemble d'administration d'oxygène selon la revendication 9, ladite au moins une paroi interne de l'interface patient empêchant le retrait du connecteur de l'évidement dans une direction orthogonale à un axe longitudinal du conduit dans la configuration raccordée, et ladite paroi d'étage nasal de l'interface patient empêchant le retrait du connecteur de l'évidement dans une direction sensiblement parallèle à un axe longitudinal du conduit dans la configuration raccordée.

11. Ensemble interface d'administration d'oxygène selon une quelconque revendication précédente, ladite première formation de raccord comprenant au moins une partie d'une surface interne de l'interface patient.

12. Ensemble interface d'administration d'oxygène selon une quelconque revendication précédente, ladite seconde formation de raccord comprenant un épaulement destiné à mettre en prise un épaulement correspondant de la première formation de raccord.

13. Ensemble interface d'administration d'oxygène selon une quelconque revendication précédente, ladite seconde formation de raccord comprenant une zone de diamètre accru par rapport à un corps principal du connecteur.

14. Ensemble interface d'administration d'oxygène selon la revendication 13, ladite seconde formation de raccord s'étendant au moins partiellement de manière annulaire autour du corps principal du connecteur.

15. Ensemble interface d'administration d'oxygène selon une quelconque revendication précédente, lesdites première et seconde formations de raccord étant raccordées de manière rotative, lors de l'utilisation.

16. Ensemble interface d'administration d'oxygène selon une quelconque revendication précédente, ladite interface patient comprenant une première formation de butée, et ledit connecteur comprenant une seconde formation de butée, de sorte que les première et seconde formations de butée coopèrent, lors de l'utilisation, pour empêcher une insertion excessive du connecteur dans l'interface patient, et ladite première et/ou ladite seconde formation de butée comprennent des saillies annulaires et/ou des évidements.
